# EUROPEAN PATENT APPLICATION

(11) **EP 3 287 149 A1**
(43) Date of publication of application: **28.02.2018**
(21) Application number: 16185130.8
(22) Date of filing: 22.08.2016
(51) Int. Cl.: A61L 15/26, C08L 77/00

(54) **WOUND DRESSING COMPRISING POLYMER FIBERS**

(71) Applicant: Friedrich-Alexander-Universität Erlangen-Nürnberg, 91054 Erlangen (DE); UNIVERSITÄTSKLINIKUM ERLANGEN, 91054 Erlangen (DE)
(72) Inventor: SCHUBERT, Dirk, Wolfram, 91330 Eggolsheim (DE); FUCHSLUGER, Thomas, 91341 Röttenbach (DE); KÜNG, Florian, 91080 Uttenreuth (DE); STAFIEJ, Piotr, 90762 Fürth (DE)
(74) Representative: Banse & Steglich

(57) **Abstract**

The present invention relates to a wound dressing, comprising a frame, which comprises at least one organic frame polymer and at least one layer of fibers spanning the frame, the fibers comprising at least one organic fiber polymer. Furthermore, the invention relates to a process for manufacturing a wound dressing of the preceding claims comprising the steps of generating at least one layer of polymer fibers comprising an organic fiber polymer by electrospinning, providing the frame which comprises an organic frame polymer, and depositing at least one layers of aligned polymer fibers on the frame.

## Description

The invention relates to wound dressings comprising a polymer frame and at least one layer of preferentially parallel aligned fibers spanning the frame. The wound dressings may be used for eye treatment, especially for the treatment of the cornea. Furthermore, a method for manufacturing of the wound dressing by electrospinning is provided.

### Background of the invention

Wound dressings are materials that serve to protect wounds from negative environmental influence, for example bacterial infections or mechanical stress and promote wound healing. In general, wounds might have different causes such as accidental mechanical injury, chemical and thermal injury or injury in the course of surgery, for example the excision of tumors.

Modern wound dressings should allow good exchange of vapor and gases, possess good absorption capacity for water and toxins, for example endotoxins or inflammation mediators, serve as a framework for infiltration and growth of cells and positively influence cell growth and wound healing.

In the field of ophthalmology, amniotic membranes are most frequently used as wound dressings and implants for treating injuries of the ocular surface, especially to promote the healing process during corneal reconstitution after injuries of the cornea. The amniotic membrane is the innermost layer of the placenta and is derived from donors undergoing cesarean section, who are negative for communicable diseases such as HIV and hepatitis. Therefore, amniotic membranes have a limited availability and are relatively costly to prepare. Furthermore, diseases might be transmitted from the donor to the patient. The amniotic membranes are usually fixed to the eye by suturing the membrane to the ocular tissue. As amniotic membranes are obtained from natural sources, the quality of the product varies and is not easy to control.

As an alternative to membranes from natural sources, artificial membranes made from naturally occurring polymers have been developed. For example, a collagen-based, suturable membrane for ocular indications is available as Visu-Foil®. However, collagen is an expensive material and is relatively quickly degraded. Additionally, the use of artificial fiber-based biocompatible and/or biodegradable materials for use as wound dressing has been evaluated. For example, polycaprolactone (PCL) membranes obtained by electrospinning have been investigated as wound dressings.

WO 2015/092797 discloses suturable multi-layer matrices comprised of biocompatible and/or biodegradable polymer fibers for use as wound dressings. Polymer fibers may, for example, be obtained by electrospinning.

Wound dressings comprising electrospun PCL membranes may additionally comprise active agents, for example the antibiotic tetracycline hydrochloride, which are released over a period of time, to promote wound healing (Chellamani KP, Vignesh Balaji RS, Veerasubramania D: Development of Wound Dressing Made of Electro Spun Tetracycline Hydrochloride Drug Incorporated PCL (Poly(E-Caprolactone)), Nanomembrane International Journal of Emerging Technology and Advanced Engineering, (2014) 4, 251 - 256).

Especially, parallel aligned fibers have raised interest for application in wound healing and tissue engineering, since they mimic the parallel aligned structure of some tissues, thus providing contact guidance and orientation to cells, to promote cell growth, tissue repair and healing.

Neither WO 2015/092797, nor Chellani et al. disclose parallel fiber structures that promote wound healing.

The generation of aligned polycaprolactone (PCL) fibers by electrospinning is, for example, disclosed by Doergens et al (Doergens A, Roether JA, Dippold D, Boccaccini AR, Schubert DW: Identifying key processing parameters for the electrospinning of aligned polymer nanofibres, Materials Letters, (2016) 140, 99-102). The document discloses the generation of PCL fibers by electrospinning and collecting the generated fibers on a rotating collector. The obtained fiber layers reveal a highly aligned orientation.

Salehi et al (Salehi S, Fathi M, Javanmard SH, Bahners T, Gutmann JS., Ergün S, Steuhl KP and Fuchsluger TA,, Generation of PGS/PCL Blend Nanofibrous Scaffolds Mimicking Corneal Stroma Structure. Macromol, Mater. Eng. (2014) 299: 455 - 469.) discloses the generation of aligned poly(glycerol sebactae (PGS)/polycaprolactone (PCL) nanofiber scaffolds by electrospinning and investigates the physical properties of these membranes for diverse tissue engineering applications, especially for cornea treatment.

US 2014/0030315 A1 discloses the use of aligned nanofiber layers from chitosan as wound dressings and suggests a use on the corneal surface.

EP 1902739 A1 discloses the use of parallel aligned biopolymer fiber layers for treating damaged ocular tissue. The aligned fiber layers are organized in a multi-layer structure, wherein the orientation in at least one fiber layer differs from that in at least a superior or inferior layer to mimic the structure of corneal tissue.

However, Salehi et al., US 2014/0030315 A1 and EP 1902739 A1 do not disclose fixing the parallel aligned fiber structure to maintain its beneficial effect. Especially in ocular use, where the fiber layers are subjected to mechanical forces by the movement of the ocular bulb and the palpebral, the parallel structures can be easily destroyed. Furthermore, no means for permanently attaching the fiber layers to the eye, for example by suturing, is disclosed.

US 2015/0024493 A1 relates to the use of aligned fiber layers for small-diameter vascular prostheses. To fix the fibers in the parallel orientation, a binding agent is applied to the aligned fibers, to bond the fibers into the aligned arrangement and to obtain aligned fiber mats. Although the technology enables fixing the parallel structure, adding a binding agent is likely to reduce porosity of the fiber layers by filling the voids between the fibers.

While wound dressings comprising parallel aligned fibers are known in the art, these wound dressings generally only have insufficient mechanical stability of the fiber layers and there are problems in fixing the biologically advantageous parallel orientation of the fibers without substantially impairing the porosity of the fiber layers. Furthermore, the wound dressings should be optimized for permanently attaching the dressings to the underlying tissue, for example to the cornea.

### Problem underlying the invention

Generally, it is a problem underlying the invention to provide dressings which overcome the above mentioned problems. Specifically, the problem is to provide a wound dressing for eye treatment, especially treatment of the cornea, having improved properties. Especially, the dressing should have good stability and the application to the patient should be convenient. More specifically, the problem underlying the invention is to provide a wound dressing, comprising at least one layer of nanofibers, which has an improved mechanical stability, wherein the improved mechanical stability enables adhering the wound dressing to a tissue by suturing or stapling. In a further aspect, the preferably parallel aligned orientation of the fiber layer shall be fixed without impairing the porous structure of the fiber layer. Furthermore, it is a problem underlying the invention to provide a simple and reproducible process for manufacturing the wound dressing.

### Disclosure of the invention

Surprisingly, it was found that the problem underlying the invention is overcome by the wound dressings and methods according to the claims. Further embodiments of the invention are outlined throughout the description.

The present invention relates to wound dressings, comprising a frame, which comprises at least one organic frame polymer and at least one layer of fibers spanning the frame, the fibers comprising at least one organic fiber polymer.

In the context of the present description, the term "fiber(s)" generally refers to polymer fiber(s).

A "fiber spanning the frame" contacts the frame in at least two sites of the frame and traverses the void surrounded by the frame between these two sites of contact.

Within the context of the present invention, the term "fiber layers" does not comprise textiles, especially no woven fabrics.

In a preferred embodiment, the wound dressing is for eye treatment, more preferably for treatment of the cornea. In the context of the present invention, the term "wound dressing" includes grafts, suitable for implantation into or onto the body, especially into or onto the eye. In a highly preferred embodiment, the wound dressing may be a graft used as artificial cornea replacement. Although the implant can be used for animals, especially mammals, in general, use for the human body and eye is highly preferred.

Preferably, the fibers in the at least one layer of polymer fibers spanning the frame are substantially parallel aligned. Within the context of the present invention, the term "substantially parallel aligned" refers to an organization of polymer fibers, wherein the longitudinal direction of less than about 40%, preferably less than about 30%, most preferably less than about 10% of the fibers in a layer deviate from the average longitudinal direction of the fibers in a layer by less than 23%, as determined by a top view on the layer of polymer fibers spanning the frame. The longitudinal direction of the fibers can be determined by analyzing the fiber layer by scanning electron microscopy and subsequent software based analysis of the obtained micrographs as for example disclosed by Doergens et al. (2015).

Preferably, the at least one layer of fibers spanning the frame is permanently adhered to the frame. The different embodiments of attaching the fiber layers to the frame are disclosed below in more detail.

As disclosed above, a parallel fiber organization promotes wound healing. By providing the layers of parallel fibers in a frame, this advantageous structure can be permanently preserved, while the porosity of the fiber layers is maintained over the whole area, where the fiber layer spans the void surrounded by the frame. Furthermore, the mechanical stability of the fiber layer is improved by the frame. Preserving the parallel structure and improving the mechanical stability is especially advantageous in applications, were the wound dressing is repeatedly subjected to mechanical forces, for example by the movement of the ocular bulb and the palpebral in ocular use of the wound dressing.

The wound dressing can be held in place during the application of the wound dressing, by holding the frame with forceps or other suitable devices. This is especially advantageous in an application wherein the dressing is applied by suturing or stapling. Consequently, the frame improves the handling of the wound dressing during application.

The term "about" could refer to a range of +/- 10%, preferably +/- 5% of the recited number or the recited number itself.

As laid out in the background section above, aligned fiber layers can be obtained by electrospinning, which will be explained in more detail below. It is therefore a highly preferred embodiment of the present invention that the polymer fiber layers spanning the frame were obtained by electrospinning.

In a further preferred embodiment of the present invention, the wound dressing comprises at least two stacked layers of fibers spanning the frame. The frame might be spanned by a stack of at least two layers of fibers on one side of the frame or on both sides of the frame. Within a stack of fiber layers, at least the fiber layer directly facing the tissue when the wound dressing is applied to a tissue, comprises substantially parallel aligned fibers. The other fiber layers comprised in the stack of fiber layers may consist of, or comprise unaligned fibers. The wound dressing may comprise about 5 to about 1000, preferably about 5 to about 100, more preferably about 5 to about 30, and most preferably 7 to 8 stacked layers of fibers spanning the frame.

The fibers in at least one fiber layer may be substantially parallel aligned in a different direction in relation to at least one other fiber layer. "Substantially parallel aligned in different direction" refers to an arrangement of the fiber layers, wherein the average longitudinal direction of the fibers in one layer deviates from the average longitudinal direction of the fibers in at least one other layer by at least more than about 10°, about 20°, about 30°, about 40°, about 50°, about 60°, about 70° and/or about 80°. By stacking or overlaying the fiber layers which are substantially parallel aligned in different directions, an overall net-like structure is obtained. This net-like structure significantly improves the mechanical stability of the aligned fiber layers.

It is an advantageous property of the described stacked arrangement of layers that the superior biological properties of aligned fiber layers are maintained, while physical stability of the wound dressing is further improved.

Preferably, the inventive wound dressing is characterized by an overall flexibility, allowing for the adaptation of the shape of the wound dressing upon application of the wound dressing to differently formed surfaces. Especially the flexibility of the wound dressing allows for an adaptation to the curved shape of the eye, especially the cornea.

In a preferred embodiment of the invention, the at least one polymer layer spanning the frame is substantially transparent, which facilitates the use of the wound dressing for cornea treatment.

In a preferred embodiment of the wound dressing according to the present invention, the frame polymer and/or the fiber polymer is a biodegradable polymer and/or a biocompatible polymer. Within the context of the present disclosure, the term "polymer" encompasses different types of polymers such as for example homo-polymers and co-polymers.

The term "biodegradable" refers to the property of a material to be able to be broken down or decomposed by microorganisms or within the body of an animal, especially when used as a wound dressing in a human, by physiological processes within a relative short time (within 1 to 18 months). Biodegradable medical devices implanted into the human body are usually not excised from the human body, but are maintained in the body until decomposition of the device under physiological conditions.

The term "biocompatible" refers to the ability of a material to perform its desired function with respect to a medical therapy, without eliciting any undesirable local or systemic effects in the recipient of the material, but generating the most appropriate beneficial cellular or tissue response. Especially, a biocompatible material is not rejected by the human immune system.

The organic frame polymer and/or the organic fiber polymer may be selected from the group consisting of, but not limited to, polyesters, polyethers, polyaminoacids, polyacrylates, acrylamide polymers, acrylate polymers, silicones, polypeptides, polysaccharides and/or polyolefins.

The organic frame polymer and/or the organic fiber polymer may for example be selected from the group consisting of, but not limited to, polymers and oligomers of glycolide, lactide, polylactic acid, polyesters of alpha-hydroxy acids, including lactic acid and glycolic acid, such as the poly(alpha-hydroxy) acids including polyglycolic acid, poly-DL-lactic acid, poly-DL-lactic acid, and terpolymers of DL-lactide and glycolide; ε-caprolactone and ε-caprolactone copolymerized with polyesters; polylactones and polycaprolactones including ε-polycaprolactone (PCL), poly(δ-valerolactone) and poly(gamma-butyrolactone); polyanhydrides; polyorthoesters; other hydroxyl acids; polydioxanone; polysaccharides, such as chitosan, and other biologically degradable polymers that are non-toxic or are present as metabolites in the body. Examples of polyaminoacids include, but are not limited to, polylysine (PLL), poly L-aspartic acid, poly L-glutamic acid, and styrene-maleic acid anhydride copolymer. Examples of derivatives of polyethylene glycol includes, but are not limited to, poly(ethylene glycol)-di-(ethylphosphatidyl) (ethylene glycol)) (PEDGA), poly(ethylene glycol)-co-anhydride, poly(ethylene glycol)co-lactide, poly(ethyleneglycol)-co-glycolide and poly(ethylene glycol)-co-orthoester. Examples of acrylamide polymers include, but are not limited to, polyisopropylacrylamide, and polyacrylamide. Examples of acrylate polymers include, but are not limited to, diacrylates such as polyethylene glycol diacrylate (PEGDA), oligoacrylates, methacrylates, dimethacrylates, oligomethoacrylates and PEG-oligoglycolylacrylates. Examples of carboxyalkyl cellulose include, but are not limited to, carboxymethyl cellulose and partially oxidized cellulose.

In an alternative embodiment of the wound dressing according to the present invention, the frame polymer and/or the fiber polymer is a biostable polymer. As used herein, "biostable" refers to polymers which are not broken down or decompose by microorganisms or within the body of an animal over an extended period of time of several months or years. Examples of biostable polymers include, but are not limited to, silicones, polyurethanes, polyethylenes, polysulfones, polyisobutylene, poly-4-methylpentene, polypropylene, polyvinylethylene, polybutylene, polydodecyl methacrylate, polyethlyene terephthalate, ethylene vinyl acetate, ethylene vinyl alcohol copolymer, polyethylene oxide, combinations thereof or copolymers thereof.

Since the main aspect of the present invention relates to the use of fibers generated by electrospinning, in a preferred embodiment, the organic fiber polymer is selected from a polymer suitable for processing by electrospinning.

Preferably, the organic frame polymer and/or organic fiber polymer is selected from the group consisting of elastin, collagen, polyurethane, ε-polycaprolactone (PCL), polylactide, polyglycolic acid, mixtures of these, and copolymers of these. These polymers are suited for electrospinning.

In general, a mixture of the above-mentioned polymers may be used in accordance with the present invention.

Most preferably, the polymer is PCL. The PCL used in the wound dressing of the present invention may have an average molecular mass of about 20 kDa to about 180 kDa, preferably from about 40 kDa to about 90 kDa, more preferably from about 70 kDa to about 85 kDa and most preferably about 80 kDa.

Different polymers may be selected as organic frame polymer or organic fiber polymer. Also, the different fiber layers may be comprised of different organic fiber polymers. However, in a highly preferred embodiment of the present invention, the organic frame polymer and the organic fiber polymer are the same polymer. It is also preferred that the different fiber layers are comprised of the same organic fiber polymer. Preferably, the wound dressing consists of one polymer material. Preferably, this one polymer is a biodegradable polymer, most preferably PCL.

It is a highly advantageous aspect of the wound dressing consisting of one biodegradable polymer, that the entire wound dressing can be degraded by the human body. Thus, no excision or detachment of the wound dressing or parts of the wound dressing is required after the wound has healed.

The fibers employed in the wound dressing of the present invention may be microfibers or nanofibers. Microfibers are fibers with an average diameter in the micrometer range. Accordingly, nanofibers are fibers with an average diameter in the nanometer range. Preferably, the fibers have an average diameter between about 100 nm and about 1,500 nm, preferably between about 200 nm and about 1,200 nm, more preferably between about 400 nm and about 1,000 nm, most preferably between about 500 nm and 900 nm.

According to the present invention, the at least one layer of fibers is porous. In the embodiment wherein several layers of fibers are stacked, it is a preferred embodiment that the stacked fiber layers also constitute an overall porous stack of layers. "Porous" layer or layers comprise voids (spaces where no polymeric material or other material is present) connecting the sides of the layers, thereby enabling an exchange of fluid or gas between the sides of the layer or layers. Thus, the overall wound dressing is porous in the axial direction, thereby allowing exchange of air and liquid between the wound and the environment.

In addition to the polymer material, the fiber layers may comprise at least one additional compound, especially at least one active agent. The at least one active agent may especially be selected from small molecular weight compounds, polypeptides and proteins.

The active agent may, for example, be a pharmaceutically active agent and/or an active agent that promote cell growth, adhesion or differentiation. In general, such active agent may improve wound healing. Preferably, the pharmaceutically active agent may be selected from agents which are typically used for ophthalmological applications, for example from anti-microbial agents, anti-bacterial agents; especially gentamicin or penicillin; anti-inflammatory agents, anti-viral agents, anti fungal agents, growth factors; for example epidermal growth factor or platelet lysate; anti scarring agents; for example rosiglitazone; substances against dry eye disease; for example hyaluronic acid, tacrolimus, diquafosol; and/or substances hindering the degradation of the extracellular matrix; for example cacicol. The active agents are released from the wound dressing under physiological conditions after the wound dressing has been applied to the wound.

In a preferred embodiment, the wound dressing comprises a hydrogel. Preferably, the hydrogel comprises at least one active agent, preferably an active agent selected as outlined above. The hydrogel may be comprised in form of at least one additional layer on top or between the layers of fibers. Alternatively, the hydrogel may be used to fill the voids of the porous structure of the wound dressing. The term "hydrogel" refers to a cross-linked network of hydrophilic polymers which are capable of absorbing high amounts of water. In particular, the cross-linked polymer hydrogels are capable of absorbing an amount of water in excess of 10 times their dry weight. The hydrogel may be a biodegradable and/or a non-biodegradable hydrogel. The polymer comprised in the hydrogel may be selected from chitosan, alginates, poly(ethylene glycole), and/or poly(ethylene oxid), which are examples for typical hydrogels used for medical applications. The hydrogel may comprise particles, including particles of at least one hydrogel different from the surrounding hydrogel or particles comprising at least one active agent. By varying the degree of polymer cross-linking, the release kinetics of active agents from the wound dressing may be varied. A higher degree of cross-linking will result in a slower release of the active agents, whereas a lower degree of cross-linking will result in a faster release of the active agents.

The frame of the wound dressing may consist of a polymer film. Alternatively, the frame may consist of randomly oriented, such as a non-woven fiber material. Non-woven fiber materials with various fiber-diameters may be obtained by known methods, including wet fiber spinning, dry fiber spinning, melt fibers spinning, melt blow fiber spinning, gel spinning and electrospinning. In some cases, the fibers may be bonded after spinning. The bonding of the fibers may be performed by thermobonding, for example with head sealers or calenders, hydroentanglement, ultrasonic pattern bonding, needlepuniching, chemical bonding, and other methods known in the art.

The size and the form of the frame may be selected depending on the desired application area of the wound dressing. Preferably, the frame has the size and form suitable for application of the wound dressing to the human eye, especially to the cornea. Most preferably, the frame has the form of a ring.

In a preferred embodiment of the invention, the shape of the wound dressing is convex and may preferably be configured to cover an average cornea, preferably a human cornea. The eye as an organ has a complex geometry with the cornea resting as a dome on top of the eyeball. The dome of the human cornea covers an elliptical area with a mean horizontal meridian of 11.7 mm and a mean vertical meridian of 11.6 mm. The radius of curvature varies between 7 mm and 8.5 mm. This macroscopic shape of the cornea is taken into account in the wound dressing configured to cover an average cornea. Especially, the inventive wound dressing may cover an elliptical area with a mean horizontal meridian of about 11.7 mm, a mean vertical meridian of about 11.6 mm, and a radius of curvature of between about 7 mm and about 8.5 mm. Advantageously, a convexly shaped wound dressing reduces the risk of folds during the application of the wound dressing to the cornea. Due the curvature fitting of the wound dressing, it is potentially possible to attach the wound dressing sutureless due to the high adhesion.

A wound dressing of the described shape may be obtained by depositing the frame and/or the polymer fibers on molds shaped like an average cornea. Furthermore, a mold based on the specific shape of the cornea of a patient may be manufactured in order to create a wound dressing with a perfect fit to the patient's eye.

In a preferred embodiment of the invention, the frame is suitable for suturing or stapling the frame to a biological tissue. Thus, the thickness and width of the frame may be selected by the person skilled in the art to satisfy this requirement. Within the context of the present application "suitable for suturing" refers to the property that a needle with a thread can pass through the frame without causing a rupture or tearing of the frame when the needle passes the frame or during the time when the thread adheres the frame to an underlying tissue. Likewise, "suitable for stapling" refers to the property that a stapler can pass through the frame without causing rupture or tear of the frame.

The advantageous suitability of the described wound dressing for suturing or stapling is facilitated by the frame of the wound dressing, which enables fixation of the wound dressing to an underlying tissue without disturbing or even destroying the structural integrity of the parallel fiber layer.

In a specific embodiment, the inner diameter of the ring is between about 3 mm and about 20 mm, and the outer diameter of the ring is between about 5 mm and about 25 mm, preferably the inner diameter is about 10 mm and the outer diameter is about 14 mm.

Preferably, the resulting width of the ring is between about 0.5 mm and about 5 mm, more preferably between about 1 mm and about 2 mm.

In a preferred embodiment of the present invention, the fibers are permanently adhered to the frame. "Permanently adhered" fibers are connected to the frame under the usual conditions of use and over the average lifetime of the wound dressing. Preferably, the fibers are adhered to the frame by means of an adhesive, by welding or most preferably by embedding the fibers in the material constituting the frame. The fibers might be adhered to the frame by applying an adhesive composition. A suitable adhesive composition can be selected by the person skilled in the art depending on the nature of the fiber polymer and the frame polymer. Welding of fibers to the frame may be achieved by heat treatment, which leads to a partial melting of the fibers and/or the frame, thereby permanently adhering frame and fibers.

In a preferred embodiment, the layer or layers comprising aligned fibers are embedded in the material constituting the frame to permanently adhere the fibers to the frame. In this specific embodiment, the organic fiber polymer and the organic frame polymer are the same polymers. The embedding of the fibers in the material constituting the frame may be achieved by subjecting the contact sites of the fibers with the frame with a suitable organic solvent comprising the respective polymer and, partially solubilizing the polymer of the fibers and the frame and partially mixing the fiber polymer and the frame polymer at the contact site. After evaporating the solvent, the fiber material is thus embedded in the material constituting the frame.

In a further aspect, the present invention relates to a kit comprising the wound dressing described above and instructions for using the dressing. Furthermore, the kit might comprise a surgical needle and/or thread to be used for applying the wound dressing.

The present invention furthermore relates to a process for manufacturing a wound dressing as described above, comprising the steps of
(a) generating at least one layer of polymer fibers comprising an organic fiber polymer by electrospinning,
(b) providing the frame which comprises an organic frame polymer, and
(c) depositing at least one layer of aligned polymer fibers on a frame.

Preferably, several layers of aligned polymers are deposited on the frame in step (c).

The generation of fibers by electrospinning is a process known in the art (Doergens et al. (2015), WO 01/27365 A1, US 2014/0046236, WO 2015/092797 A1). As used herein, the term "electrospinning" refers to a technology which produces fibers from a polymer solution or melt. Preferably, the fibers are generated from a polymer solution. In the process, the polymer solution or melt, is placed in a dispenser, and the generated fibers are collected by a collector. The dispenser is connected to a source of high-voltage, preferably of positive polarity, while the collector is grounded. Thereby an electrostatic filed is induced between the dispenser and the collector. At a critical voltage, the charge repulsion begins to overcome the surface tension of the solution or melt, generating a jet that travels from the dispenser to the collector. While the jet travels from the dispenser to the collector, the organic solvent evaporates from the polymer solution, generating a solid fiber. Likewise, a polymer melt cools and thereby solidifies when traveling from the dispenser to the collector. The dispenser may, for example, be a syringe with a metal needle.

Aligned fibers may be obtained by using a rotating collector, for example a rotating mandrel, a rotating disk or non-solid rotating devices such as a rotating circle with copper wires or metal frame collectors. Thus, it is a preferred embodiment of the process that during electrospinning, the fibers are collected on a rotating device. In a further preferred embodiment, a layer of aligned fibers is generated by collecting the electrospun fibers on a rotating device. Preferably an electrospinning apparatus which includes a rotating device as disclosed in Doergens et al. (2015) is used. The electrospinning apparatus disclosed by Doergens et al. (2015) comprises a syringe pump with an adjustable pump rate, a syringe with an outlet consisting of a conductive material, a high voltage source and a collector which consists at least partially of a conductive material. The syringe or capillary and the collector are connected via the high voltage source.

The employed solvent may be a mixture of different organic solvents. Preferably, a mixture of chloroform and ethanol (9:1 v/v) is used. Alternatively, a mixture of di-chloromethane (DCM) and methanol (7:3 v/v) may be used.

Preferably, a solution of about 10% to about 20% by weight, more preferably about 12% to about 16% by weight, most preferably about 14% by weight of PCL is used.

The properties of the obtained fibers are influenced, amongst other factors, by the employed polymer, employed solvent, concentration of polymer solution, the applied voltage, the distance between dispenser and collector and the tangential rotation speed of the rotating collector.

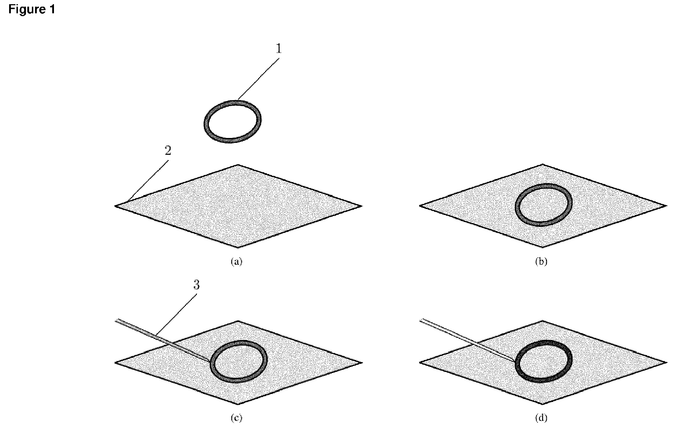

The generation of aligned polymer nanofibers by electrospinning is disclosed in detail by Doergens et al. (2015). Specific reference is made to sections 2.1. to 2.2. and Figure 1., which disclose a process and a suitable apparatus for electrospinning PCL fibers.

The frame provided in step (b) of the inventive process may be obtained by casting a film of organic frame polymer, preferably PCL, and excising the frame, preferably in the form of a ring, from said casted film.

In a preferred embodiment of the inventive process, in step (c) the at least one layer of fibers is deposited on the frame by moving the frame through the layer of fibers generated by electrospinning. It is understood that moving the frame through the layer of fibers can be achieved either by actively moving a frame through a stationary fixed layer of fibers or actively moving a layer of fibers through a stationary fixed frame.

To enable a more convenient handling of the frame in step (c) of the process described above, prior to depositing layers of aligned fibers on the frame, the frames may be placed on a flat substrate. In an alternative preferred embodiment, the substrate may be in the form of a mold comprising at least one convex dome in form of a cornea as described above. Preferably, the mold comprises an array of several domes. Preferred substrates are polytetrafluorethylene (PTFE) sheets or glass. Subsequently, an evaporable liquid, preferably water, may be dispensed between the frame and the substrate to adhere the frame on the substrate by adhesive forces.

To obtain a frame, which is adopted for the manufacture of a convex wound dressing, a two-part mold may be used. The upper part of the mold comprises a concave dome, whereas the lower part of the mold comprises a corresponding convex dome. The frame polymer in form of a film or a non-woven fabric is moved between both parts of the mold and the mold is shut. Either by pressure, temperature or by a solvent atmosphere the film or a non-woven fabric is shaped according to the geometry of the mold. The step of shaping the frame may be performed either before or after the frame is excised from the film or non-woven fabric. For example, the dome may have a radius of 8 mm while the covered area is a circle with a radius of 7 mm. The radius and area may be adjusted to the specific geometry of a patient's cornea in order to obtain a wound dressing which fits the cornea.

The deposition of several layers of fibers on the frame may be achieved by repeatedly moving the frame through the layer of fibers. To deposit different layers of fibers in different alignment orientations on the frame, the frame might be tangentially rotated between the different rounds of depositing fiber layers on the frame.

After step (c), the process may further comprise a step of cutting the fibers, which radially extend over the edge of the frame. Prior to cutting the fibers might be intermediately adhered to the frame by wetting the fibers.

In a further preferred embodiment, the inventive process furthermore comprises step d) of permanently adhering the fibers to the frame. The fibers might be permanently adhered to the frame by subjecting an adhesive composition to the contact site between fibers and frame or by welding the fibers to the frame. In a preferred embodiment, the fibers are permanently adhered to the frame by applying a solution of the fiber polymer solubilized in a solvent to the surface of the frame and subsequently evaporating the solvent. By subjecting the solution to the contact site of the frame and the fibers, the fibers and/or the frame may be partially solubilized. The choice of the organic solvent depends on the nature of the selected fiber polymer, for PCL chloroform is preferentially used. In a preferred embodiment of the process, wherein the fiber polymer is PCL, the polymer solution applied to the frame is a solution of PCL in an organic solvent. Preferably, the polymer solution has a viscosity of about 1 to about 100 Pas, preferably about 10 to about 50 Pas, most preferably about 20 Pas.

After permanently adhering the fibers to the frame, the frame may be detached from an underlying substrate by evaporating the liquid between the frame and the substrate.

A process for manufacturing a wound dressing according to the present invention is furthermore disclosed in Example 1.

In a further aspect, the present invention relates to a wound dressing obtained or obtainable by the process as described above.

The inventive wound dressing may be sterilized prior to packaging and/or use.

In a further aspect, the present invention relates to the use of the wound dressing as described above for eye treatment and/or eye surgery, preferably of the cornea. Preferably, the use comprises suturing or stapling the wound dressing to a tissue, preferably a wound or to the cornea through the frame of the wound dressing.

When used in accordance with the present invention, the wound dressing may be withdrawn from the wound or cornea after a period of time. Preferably, the wound dressing is not withdrawn from the wound or the cornea and biodegraded on the wound or cornea after a period of time.

In a further aspect, the present invention relates to a method of treatment comprising the step of applying the wound dressing as described above to a tissue, preferably a wound tissue or a cornea of an animal, more preferably a human, most preferably a patient in need of a wound treatment. Preferably, the method of treatment comprises suturing or stapling the wound dressing to a tissue, preferably a wound tissue or to the cornea through the frame of the wound dressing.

Exemplified embodiments of the invention and aspects of the invention are shown in the figures.

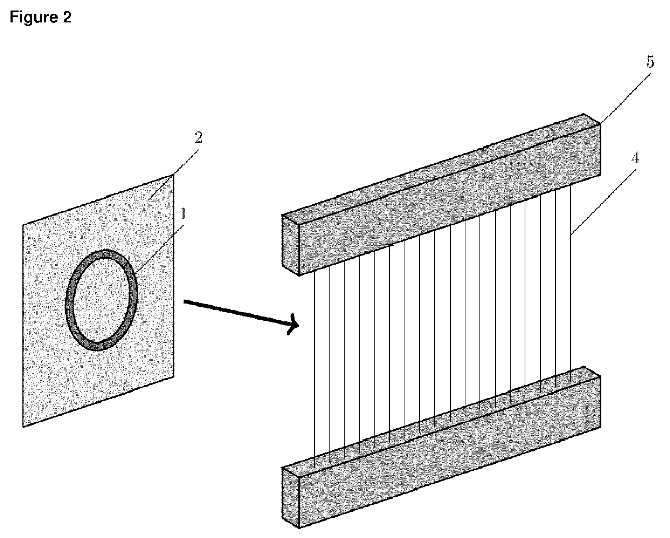

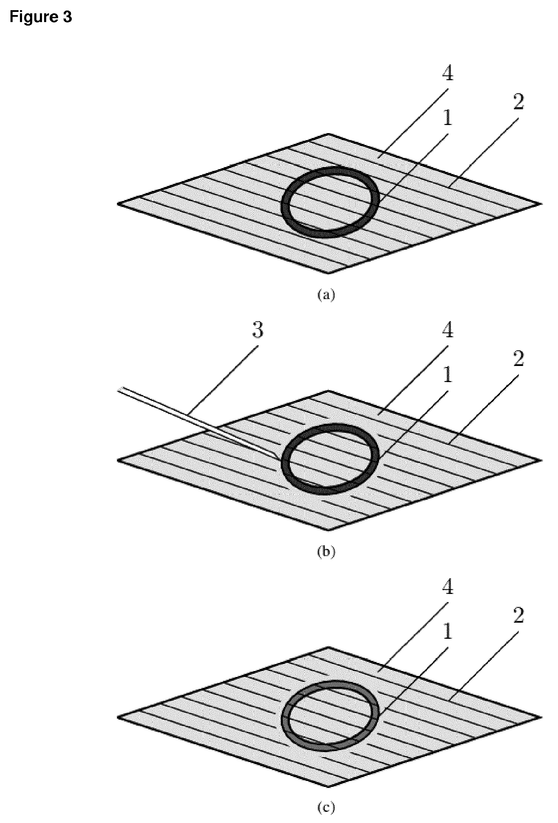

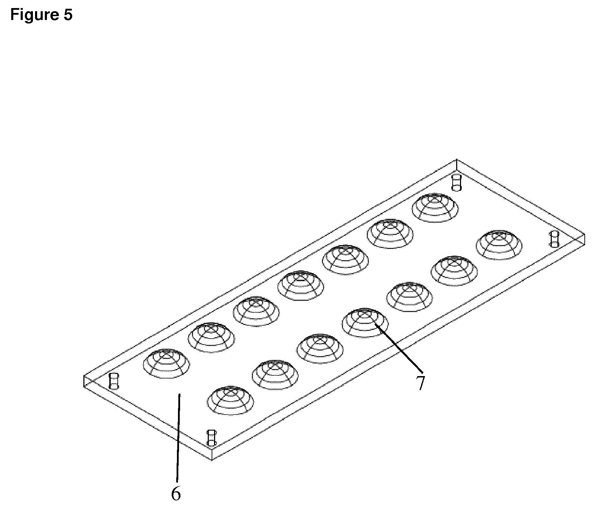

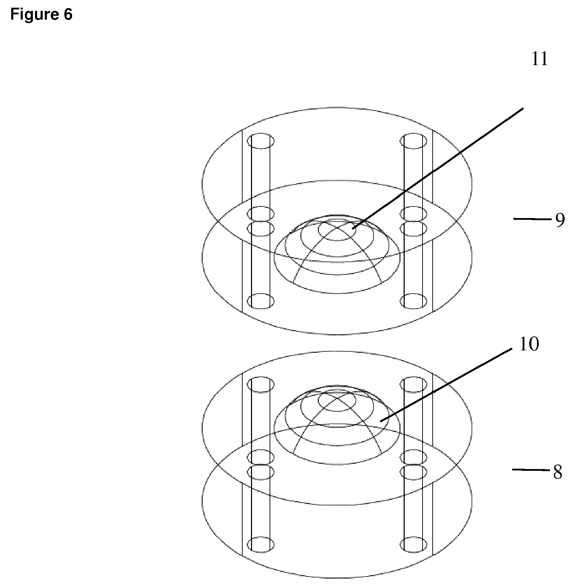

Figure 1 schematically depicts the deposition of a polymer frame in the form of a ring (1) on a flat substrate (2). By adding a liquid between the ring (1) and the substrate (2) by means of a capillary (3), the ring (1) is attached to the substrate (2) by adhesive forces. As shown in Figure 2, the ring (1) attached to the substrate (2) is moved through a layer of substantially parallel fibers (4), to deposit the fiber (4) layer on the ring (1). As described above, the fibers (4) were previously generated by electrospinning and collected on a rotating collector (5) to obtain a layer of substantially parallel fibers (4). After deposition of the substantially parallel fibers (4) on the ring (1) attached to the substrate (2), fibers (4) extending over the edge of the ring (1) are carefully cut using a capillary (3), as shown in Figure 3. As shown in Figure 5, the substrate may also be a mold (6) comprising an array of domes (7). To obtain rings that are adapted for producing convex wound dressings, a combination of a mold comprising a lower part (8) with a convex dome (10) and an upper part (9) with a concave dome (11), as depicted in Figure 6, may be used.

The wound dressing according to the invention and the process for manufacturing the wound dressing solves the problem underlying the invention. By providing fiber layers in a frame and stacking the fiber layers, the mechanical stability of the wound dressing is substantially improved, while the porosity of the fiber layers is maintained. Furthermore, the advantageous structure of substantially parallel fiber layers, which promotes wound healing, is preserved. Preserving the parallel structure and improving the mechanical stability is especially advantageous in applications, were the wound dressing repeatedly subjected to mechanical forces, for example by the movement of the ocular bulb and the palpebral in ocular use of the wound dressing.

In addition, the frame comprised in the inventive wound dressing advantageously supports the fixation of the wound dressing to an underlying tissue by suturing or stapling, without disturbing or even destroying the structural integrity of the parallel fiber layer. Furthermore, the wound dressing can be held in place during the application of the wound dressing, especially if the dressing is applied by suturing or stapling, by holding the frame with forceps or other suitable devices. Consequently, the frame improves the handling of the wound dressing during application.

It is a further advantageous property of the inventive wound dressing, that the entire wound dressing can be degraded by the human body, rendering the excision of the wound dressing or parts of the wound dressing after healing unnecessary.
Figure 1 shows schematically the attachment of a polymer frame in form of a ring to a substrate using the adhesive force of water. (a) PCL ring and substrate; (b) PCL ring on substrate; (c) Capillary with water on the PCL ring; (d) Empty capillary and attached PCL ring.
Figure 2 shows schematically the deposition of the aligned fibers on the frame.
Figure 3 shows schematically the cutting of fibers and release of the PCL rings. (a) Substrate and ring after collecting fibers; (b) Cutting the fibers with the capillary; (c) Dried PCL ring with cut fibers.
Figure 4 shows micrographs of the fibers deposited on the ring.
Figure 5 shows a mold with an array of domes for the deposition of polymer fiber layers.
Figure 6 shows a two-part mold for the production on frames adopted to the cornea.

### Examples

### Example 1: Preparation of ring shaped wound dressing with aligned PCL fibers

### 1.1 Film cast

0.6 g of polycaprolactone (PCL) (Mn of 80.000) were dissolved in 10 ml chloroform (ACS grade) and stirred with 350 U/min in a fume cupboard for 2 hours at room temperature. Petri dishes with a diameter of 9 cm were cleaned using isopropanol (technical grade) and dust free tissues. The stirred solution was cast in the petri dishes in the fume cupboard and the glass covers of the petri dishes were placed on the top. After 48 hours in the fume cupboard, the glass covers were removed and the PCL films were removed from the petri dishes.

### 1.2 Ring Preparation

The PCL films were placed on a cutting-board. Using a set of two circular knives with the diameters of 14 mm and 10 mm, rings were cut out of the PCL films.

### 1.3 Fiber Spinning

A solution of 9 ml chloroform, 1 ml ethanol (ethanol 99% denatured with 1% MEK technical grade) and 1.4 g PCL was prepared and stirred with 350 U/min in a fume cupboard for 2 hours at room temperature. The solution was filled in a 10 ml all-glass syringe with Luer-Lock™ metal nozzle. A stainless steel needle with a diameter of 0.8 mm and a flat tip was attached to the syringe using the Luer-Lock™. The remaining air in the syringe was removed by carefully releasing it through the needle.

The used electrospinning device consisted of a syringe pump with an adjustable pump rate, a high voltage source and a collector. In order to generate a high output of oriented fibers, a spinning wheel collector was chosen. The spinning wheel collector consisted of 8 metal bars held by two disks. The distance between from each bar to the next bar was 6 cm. The disks and so the bars could be rotated with up to 1000 U/min. The electrospinning device used in the experiment is disclosed in Figure 1 of Doergens et al. (2015).

The syringe was placed and fastened in the syringe pump of the electrospinning device. Using a measuring tape, the pump was positioned inside the electrospinning device so the tip of the needle was in a distance of 15 cm to the outer edge of the bars of the collector. By attaching the alligator clamp attached connected to the end of the cable coming from the high voltage source to the Luer-Lock™, the needle was connected with the high voltage source. A dispensing rate of 4 ml/h was set on the syringe pump and a voltage of 20 kV on the high voltage source. The collector was set on a rotating rate of 900 U/min. After one hour of electrospinning the collector was stopped, the voltage was removed and the syringe pump was turned off.

### 1.4 Fiber Harvesting

The PCL rings were placed next to each other on a flat substrate (A PTFE sheet with the thickness of 2.0 mm or a glass substrate for microscopy were used). Using a syringe, a little amount of water (on the 10 µl scale) was dispensed between the substrate and the ring in order to make the ring adhere on the substrate by using reversible adhesive forces. The process of attaching the rings to the substrate is shown in Figure 1.

Some fibers at the side of the collector were destroyed in order to generate an access point for the substrate. The substrate was picked up with the tweezers and moved behind the fibers. By moving the substrate through the fibers, as shown in Figure 2, the fibers were harvested and deposited on the rings. The process of collecting the fibers can be done repeatedly on several locations on the collector in order to generate thicker layers of fiber. If desired, the substrate can be rotated between different collection steps in order to generate layers comprising substantially parallel fibers which are oriented in different directions in the different layers.

After depositing the parallel orientated fibers on the PCL rings and the substrate, the substrate was put down with the rings facing upwards. Using a syringe, the fibers close to the outer side of the ring were wetted. The fibers extending over the edge of the rings were carefully cut using either the sharp tip of the capillary or a scalpel. The rings were left on the substrate until the water had evaporated (about 30 minutes), subsequently the rings were removed from the substrate using tweezers. The progress of freeing the PCL rings is schematic shown in Figure 3.

### 1.5 Optional: Connecting the rings and the fibers

In order to generate a more stable connection between the ring and the fibers, a PCL and chloroform solution with a viscosity of about 20 Pas (range of 1.0 Pas to 100.0 Pas) was prepared. A capillary was drawn from a Pasteur pipette using the flame of a Bunsen burner. The solution was carefully applied on top of the surface of the PCL ring covered with fibers. The fibers resting on the surface of the PCL ring and the surface of the PCL ring were partial dissolved. After the evaporation of the solvent, the ends of the fibers were embedded in the PCL ring.

### 2 Results

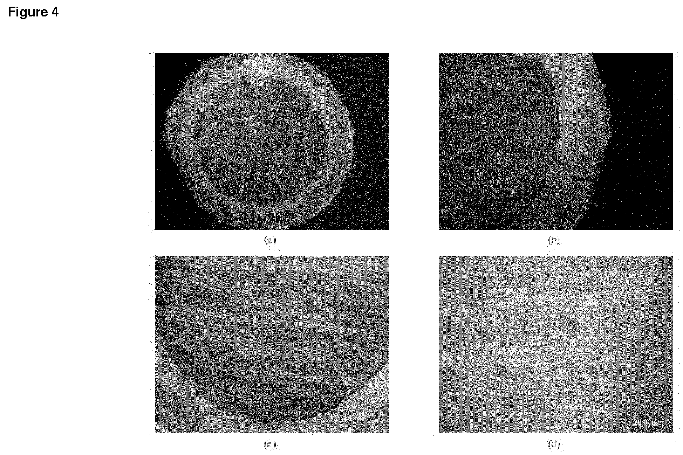

By the described procedure, a wound dressing as shown in Figure 4 with different magnifications was obtained. The wound dressing contains several layers of aligned fibers deposited in different orientations. The beads on the fibers are a result of the electrospinning process. The generation of said beads can be avoided by optimizing the conditions of the electrospinning process.

The wound dressing obtained by the process as described in Example 1 exhibits a high mechanical stability and a high porosity in the axial direction of the wound dressing, which allows exchange of gas and fluids. Furthermore, the advantageous structure of substantially parallel aligned fibers, which promotes wound healing, is fixed by the permanent attachment of the fiber layers to the frame. The polymer frame of the wound dressing advantageously supports the fixation of the wound dressing to an underlying tissue by suturing or stapling, without disturbing or even destroying the structural integrity of the parallel fiber layer.

## Claims

1. A wound dressing, comprising a frame (1), which comprises at least one organic frame polymer and at least one layer of fibers (4) spanning the frame (1), the fibers (4) comprising at least one organic fiber polymer.

2. The wound dressing according to claim 1, wherein the fibers (4) in the at least one layer are substantially parallel aligned.

3. The wound dressing according to any of the preceding claims, wherein the fibers were obtained by electrospinning.

4. The wound dressing according to any of the preceding claim, which comprises several layers of fibers (4) spanning the frame, wherein the fibers are substantially parallel aligned in different directions in different layers.

5. The wound dressing according to any of the preceding claims, wherein
the frame polymer and/or the fiber polymer is a biodegradable polymer; and/or
the frame polymer and/or the fiber polymer is selected from the group consisting of polyester, polyether, polyaminoacid, polyacrylate, acrylamide polymers, acrylate polymers, silicone, polypeptide, polysaccharide and/or polyolefin, wherein preferably the frame polymer and/or the fiber polymer is selected from elastin, collagen, polyurethane, ε-polycaprolactone (PCL), polylactide, and/or polyglycolic acid; and/or the frame polymer and the fiber polymer are the same polymers.

6. The wound dressing according to any of the preceding claims, wherein the at least one layer of fibers (4) is porous.

7. The wound dressing according to any of the preceding claims, wherein the wound dressing comprises a hydrogel, preferably a hydrogel comprising at least one active agent.

8. The wound dressing according to any of the preceding claims, wherein the frame (1) consists of a polymer film or a non-woven fiber material, preferably in the form of a ring.

9. The wound dressing according to any of the preceding claims, wherein the shape of the wound dressing is convex, preferably configured to cover an average cornea.

10. The wound dressing according to any of the preceding claims, wherein the at least one layer of fibers spanning the frame is permanently adhered to the frame.

11. The wound dressing according to any of the preceding claims, wherein the layer or layers of fibers (4) spanning the frame (1) are embedded in the material constituting the frame (1).

12. The wound dressing according to any of the preceding claims, wherein the wound dressing is for eye treatment, preferably for treatment of the cornea, and/or eye surgery, preferably for surgery of the cornea.

13. The process for manufacturing a wound dressing of the preceding claims comprising the steps of
(a) generating at least one layer of polymer fibers (4) comprising an organic fiber polymer by electrospinning,
(b) providing the frame (1) which comprises an organic frame polymer, and
(c) depositing at least one layer of aligned polymer fibers (4) on the frame (1),
wherein optionally in step c) the layer of polymer fibers (4) is deposited on the frame (1) by moving the frame (1) through the layer of fibers (4) generated by electrospinning,
and optionally further comprising step
(d) of permanently adhering the fibers (4) to the frame.

14. A process according to claim 13, wherein the polymer fibers (3) comprise polycaprolactame and the polymer solution is a solution of polycaprolactame in an organic solvent.

15. The wound dressing obtained or obtainable by the process of any of claims 13 or 14.
